(19) Europäisches Patentamt
European Patent Office
Office européen des brevets

(11) Publication number: 0 188 214
A2

# (12) EUROPEAN PATENT APPLICATION

(21) Application number: 86100127.9

(22) Date of filing: 07.01.86

(51) Int. Cl.⁴: C 07 K 7/00
A 61 K 37/02

(30) Priority: 17.01.85 US 692082

(43) Date of publication of application: 23.07.86 Bulletin 86/30

(84) Designated Contracting States: AT BE CH DE FR GB IT LI LU NL SE

(71) Applicant: ADMINISTRATORS OF THE TULANE EDUCATIONAL FUND
St. Charles Avenue
New Orleans Louisiana 70118(US)

(72) Inventor: Coy, David Howard
4319 Perrier Street
New Orleans Louisiana 70115(US)

(72) Inventor: Murphy, William Andrew
601 West Hall Avenue
Slidell Louisiana 70458(US)

(74) Representative: Wächtershäuser, Günter, Dr.
Tal 29
D-8000 München 2(DE)

## (54) Novel growth hormone-releasing peptides and method of treating mammals therewith.

(57) The present invention relates to novel growth hormone-releasing peptides and method for increasing the release of growth hormone levels in mammals, as well as method for increasing milk production in dairy cows and increasing growth rates of animals, such as cattle, sheep, swine, and others. The novel peptides of this invention surprisingly are effective for releasing growth hormone levels in animals by replacing the natural amino acids in positions 1, 2, 3, 8, 9, 10, 12, 21 and 27, as well as derivatizing the N-terminal amino acid residue in human pancreatic islet tumor origin growth hormone-releasing factor.

EP 0 188 214 A2

# NOVEL GROWTH HORMONE-RELEASING PEPTIDES AND METHOD OF TREATING MAMMALS THEREWITH

This application is a continuation-in-part of application Serial No. 692,082, filed January 7, 1985, which is a continuation-in-part of application Serial No. 605,520, filed April 30, 1984, which is a continuation-in-part of application Serial No 522,067, filed August 10, 1983.

The present invention relates to novel growth hormone-releasing peptides and method for increasing the release of growth hormone levels in mammals therewith. Further, methods for increasing milk production in dairy cows result when the novel growth hormone-releasing peptides of the present invention are administered to those cows.

Recently, growth hormone-releasing factors (GRF) of human pancreatic islet tumor origin (hpGRF) were isolated, characterized, and shwon to possess growh hormone(GH)-releasing activity in rat anterior pituitary in vitro and in vivo by (1) Guillemin, P. Brazeau, P Böhlen, F. Esch, N. Ling, and W. B. Wehrenberg [Science, 218, 585 (1982)] and (2) J. Spiess, J. Rivier, M. Thorner, and W. Vale [Biochemistry, 21, 6037 (1082)]. Further, a synthetic hp GRF(]-29)-$NH_2$, an amidated fragment of the natural hp GRF(1-29)-$NH_2$, an amidated fragment of the natural hp GRF, was reported to possess full intrinsic biological activity by Spiess et al. of reference (2).

AMINO ACID SEQUENCE IN DESIGNATED PEPTIDES

HPGRF (1-44)$NH_2$

(Guillemin) Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-I13-Met-Ser-Arg-Gln-Gln-Gly-Glu-Ser-Asn-Gln-Glu-Arg-Gly-Ala-
29

Arg-Ala-Arg-Leu-$NH_2$;
44

hpGRF (1-40)

(Vale) Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-Ile-Met-Ser-Arg-Gln-Gln-Gly-Glu-Ser-Asn-Gln-Glu-Arg-Gly-Ala;
29 40

hpGRF (1-29)$NH_2$

Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-Ile-Met-Ser-Arg-$NH_2$.
29

Code for L-Amino Acid, per IUPAC-IUB Commission on Biochemical Nomenclature:
Biochemistry 11, 1726-1732 (1972)

| | | | |
|---|---|---|---|
| His = Histidine | Val = Valine | Tyr = Tyrosine | Gln = Glutamine |
| Ser = Serine | Phe = Phenylalanine | Arg = Arginine | Met = Methionine |
| Asp = Aspartic Acid | Thr = Threonine | Leu = Leucine | Ile = Isoleucine |
| Ala = Alanine | Asn = Asparagine | Lys = Lysine | Glu = Glutamic Acid |
| Gly = Glycine | | Nle = Norleucine | |

Since these hpGRF peptides contain 29 to 44 amino acid units with their molecular weights ranging from 3,085 to 5,035 Daltons, it is desirable to increase their potency so that the dosages administered for practical purpose could be reduced for eliciting the release of suitable levels of GH in mammals. It is, therefore, an object of the present invention to provide more potent peptides which mimic hpGRF and are effective for increasing the release of growth hormone to suitable levels in mammals.

Surprisingly, this objective is achieved by replacing the natural amino acid residues in positions 1, 2, 3, 8, 9, 10, 12, 21 and 27, as well as derivatizing the N-terminal amino acid residue in hpGRF 1 to 29, 1 to 40 and 1 to 44 as expressed below by the structural formulas (I), (II), and (III) respectively; provided that at least one of two natural amine acid residues in the 2,9, 10, 12 or 21 positions of the hpGRF 1 to 29, 1 to 40 or 1 to 44 is replaced in the 2 position with N-methylalanyl or N-methyl-D-alanyl; in the 9 position with D-seryl; in the 10 position with D-tyrosyl; or in the 12 and 21 positions with arginyl. These achievements are unexpected especially in view of reports by N. Ling and P. Brazeau [The Endocrine Society Program and Abstracts, 65th Annual Meeting, No. 295, June 8 to 10, 1983, at San Antonio, Texas] which indicate that AC-$Tyr^1$ and D-$Tyr^1$ hpGRF analogs have low potency with respect to the natural peptide, and by J. Rivier et al. [Abstract 8th American Peptide Symposium, May 22-27, 1983, paper 10-B], who indicated for hpGRF (1-27) that "some manipulation of the Tyr residue is compatible as long as a free amino terminus is conserved."

## DESCRIPTION OF THE INVENTION

The polypeptides of the present invention are depicted by the structures of formulas (I), (II), and (III) below:

$R^1$-A-B-C-Ala-Ile-Phe-Thr-X-Q-Z-Arg-U-Val-Leu-
1 10
Gly-Gln-Leu-Ser-Ala-Arg-W-Leu-Leu-Gln-Asp-Ile-Y-
20
Ser-Arg-$R^2$
29

(I)

$R^1$-A-B-C-Ala-Ile-Phe-Thr-X-Q-Z-Arg-U-Val-Leu-
1
Gly-Gln-Leu-Ser-Ala-Arg-W-Leu-Leu-Glu-Asp-Ile-Y-
Ser-Arg-Gln-Gln-Gly-Glu-Ser-Asn-Gln-Glu-Arg-Gly-Ala-$R^2$
29 40

(II)

$R^1$-A-B-C-Ala-Ile-Phe-Thr-X-Q-Z-Arg-U-Val-Leu-
1
Gly-Gln-Leu-Ser-Ala-Arg-W-Leu-Leu-Gln-Asp-Ile-Y-
Ser-Arg-Gln-Gln-Gly-Glu-Ser-Asn-Gln-Glu-Arg-Gly-Ala-
29
Arg-Ala-Arg-Leu-$R^2$
44

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl,

D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

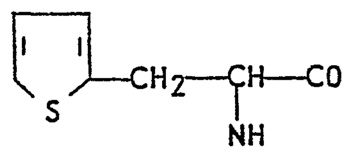 ;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

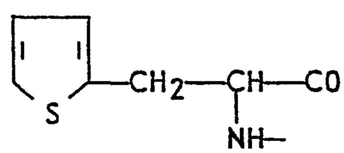 ; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or u and w are each arginyl; and the pharmaceutically acceptable salts thereof.

Preferred compounds of the invention are represented by Formulas (I), (II), or (III) wherein $R^1$, $R^2$, $R^3$, and $R^4$ are as described above; and A is selected from tyrosyl, D-tyrosyl, histidyl, D-histidyl; B is selected from alanyl, D-alanyl, N-methylalanyl, N-methyl-D-alanyl, leucyl, D-leucyl; C is selected from aspartyl, D-aspartyl, glutamyl and D-glutamyl; Q is seryl or D-seryl; X is selected from asparaginyl or D-asparaginyl; Y is selected from norleucyl or methionyl; U is lysyl or arginyl; and W is lysyl or arginyl; Z is tyrosyl or D-tyrosyl; with the above-mentioned proviso; and the pharmaceutically acceptable salts thereof.

Another preferred group of compounds of the invention have the Formula (I) configuration, wherein $R^1$ is seleted from hydrogen or $C_1$-$C_3$ alkanoyl; $R^2$ is $NH_2$ or OH; A is selected from tyrosyl, D-tyrosyl or histidyl; B is alanyl or D-alanyl; C is aspartyl or D-aspartyl; Q is seryl or D-seryl; U is arginyl; W is arginyl; X is asparaginyl or D-asparaginyl; Y is norleucyl or methionyl, and Z is tyrosyl or D-tyrosyl; and the pharmaceutically acceptable salts thereof.

The most preferred group of compounds of the invention have the formula (I) configuration, wherein $R^1$ is selected from hydrogen or acetyl. $R^2$ is $NH_2$ or OH; A is selected from tyrosyl, D-tyrosyl or histidyl; B is alanyl or D-alanyl; C is aspartyl or D-aspartyl; Q is seryl or D-seryl; U is arginyl; W is arginyl; X is asparaginyl or D-asparaginyl; and Y is norleucyl or methionyl, and Z is tyrosyl or D-tyrosyl; and pharmaceutically acceptable salts thereof.

The term "pharmaceutically acceptable salts" as used herein refers to non-toxic alkali metal, alkaline earth metal, ammonium, organoammonium and metallic salts commonly used in the pharmaceutical industry. These salts include, but are not limited to, the sodium, potassium, lithium, calcium, magnesium, zinc, ammonium, and trimethylammonium salts which are prepared by methods well

known in the art. The term also includes non-toxic acid addition salts such as hydrochloride, hydrobromide, acetate, phosphate, sulfate, citrate, laurate, stearate, palmoate, and oleate, but are not limited to them. These acid addition salts are also prepared by methods well known in the art.

Further, the term "organoammonium" is defined as a group consisting of a positively charged nitrogen atom joined to from one to four aliphatic groups, each containing from one to 20 carbon atoms. Among the organic ammonium groups which are illustrative for the preparation of the aliphatic ammonium salts of this invention are: monoalkylammonium, dialkylammonium, trialkylammonium, tetraalkylammonium, monoalkenylammonium, dialkenylammonium, trialkenylammonium, monoalkynylammonium, dialkynylammonium, trialkanolammonium, $C_5$-$C_6$ cycloalkylammonium, piperidinium, morpholinium, pyrrolidinium, benzylammonium, and equivalents thereof.

In keeping with the standard nomenclature, abreviations for chiral amino acid residues used in the present specification and claims are as follows:

| Abbreviation | Name |
|---|---|
| His | L-histidyl |
| Ser | L-seryl |
| D-Ser | D-Seryl |
| Asp | L-aspartyl |
| D-Asp | D-aspartyl |
| Ala | L-alanyl |
| D-Ala | D-alanyl |
| Val | L-valinyl |
| Phe | L-phenylalanyl |
| Thr | L-threonyl |
| Asn | L-asparaginyl |
| D-Asn | D-asparaginyl |
| Tyr | L-tyrosyl |
| Arg | L-arginyl |
| Leu | L-leucyl |
| D-Leu | D-leucyl |
| Nle | L-norleucyl |
| Lys | L-lysy l |
| Gln | L-glutaminyl |
| Met | L-methionyl |
| Ile | L-isoleucyl |
| Glu | L-glutamyl |
| D-Glu | D-glutamyl |
| D-Tyr | D-tyrosyl |
| D-His | D-histidyl |
| D-Phe | D-phenylalanyl |
| (4-Cl)Phe | L-4-chlorophenylalanyl |
| D-(4-Cl)Phe | D-4-chlorophenylalanyl |
| (4-Br)Phe | L-4-bromophenylalanyl |
| D-(4-Br)Phe | D-4-bromophenylalanyl |
| (4-F)Phe | L-4-fluorophenylalanyl |
| D-(4-F)Phe | D-4-fluorophenylalanyl |
| (4-MeO)Phe | L-4-methoxyphenylalanyl |
| D-(4-MeO)Phe | D-4-methoxyphenylalanyl |

| Abbreviation | Name |
|---|---|
| (4-ØCH2O)Phe | L-4-benzloxyphenylalanyl |
| D-(4-ØCH2O)Phe | D-4-benzloxyphenylalanyl |
| Trp | L-tryptophyl |
| D-Trp | D-tryptophyl |
| (5-F)Trp | L-5-fluorotryptophyl |
| D-(5-F)Trp | D-5-fluorotryptophyl |
| (5-Cl)Trp | L-5-chlorotryptophyl |
| D-(5-Cl)Trp | D-5-chlorotryptophyl |
| (5-Br)Trp | L-5-bromotryptophyl |
| D-(5-Br)Trp | D-5-bromotryptophyl |
| (5-MeO)Trp | L-5-methoxytryptophyl |
| D-(5-MeO)Trp | D-5-methoxytryptophyl |
| (5-Me)Trp | L-5-methyltryptophyl |
| D-(5-Me)Trp | D-5-methyltryptophyl |

Unless otherwise specified, the amino acid residues that are named herein without the prefix L will refer to the naturally occurring absolute configuration L. The $R^1$ group refers to the substituent on the N-terminus amino acid (position 1 of the peptide according to standard nomenclature.

Other abbreviations used in the present specification are:

| | | |
|---|---|---|
| Fmoc | = | fluorenylmethyloxycarbonyl |
| Boc | = | t-butyloxycarbonyl |
| Tos | = | p-toluenesulfonyl |
| hplc | = | high performance liquid chromatography |
| tlc | = | thin-layer chromatography |
| TFA | = | trifluoroacetic acid |
| Ac | = | acetyl |
| Z | = | benzyloxycarbonyl |

Solid-phase synthesis of the Formulas (I), (II), and (III) peptides can be carried out on a Beckman 990 automatic peptide synthesizer. Preparative HPLC can be performed on a thick-walled glass column (2.5 x 45 cm) containing Whatman LRP-1 reverse phase packing ($C_{18}$ silica 13-22 μm) pumped with Fluid Metering Company pump and pulse damper. Amino acid analyses can be run on a Beckman 119 CL analyzer and processed with a System AA computing integrator.

Amino acid derivatives utilized in the preparation of the compounds of the present invention are available from several chemical supply houses including: Bachem, Inc., Torrance, California, and Chemical Dyanamics, Inc., Plainfield, New Jersey.

The peptides having the Formulas (I), (II), and (III) configurations can be conveniently prepared by standard solid-phase techniques; for example, the C-terminal protected amino acid can be attached to a chloromethyl resin, a hydroxymethyl resin, a benzhydrylamine (BHA) resin or a p-methylbenzylhydrylamine (p-Me-BHA) resin. One such chloromethyl resin is sold under the trade name Bio-Beads SX-1 by Bio Rad Laboratories, Richmond, California. The preparation of the hydroxymethyl resin is described by Bodansky et al., Chem. Ind. (London) 38, 1597 (1966). The BHA resin has been described by Pietta and Marshall, Chem. Commun. 650 (1970) and commercially available from Bachem, Inc.. Torrance, California.

According to an embodiment of the invention, the peptides of Formulas (I), (II), and (III) are prepared by means of solid-phase peptide synthesis by standard procedures, although it may also be prepared by treatment of the peptide-resin with ammonia to give the desired side-chain protected amide or with an alkylamine to give a side-chain protected alkylamide or dialkylamide.

The α-amino protecting group is Fmoc for the amino acid in position one, and the side-chain protecting group is Boc instead of Z for the appropriate preceeding amino acid, when the chloromethyl or hydroxymethyl resin is used.

Side-chain protection can then be removed in the usual fashion by treatment with HF to give the free peptide amides, alkylamides, or dialkylamides.

In preparing the esters of this invention, the resins used to prepare the acids of Formulas (I), (II), and (III) ($R^2$ = OH) can be employed, and the side-chain protected peptide can be cleaved with a base and appropriate alcohol, i.e., methanol. Side-chain protecting groups can then be removed in the usual fashion by treatment with HF to obtain the desired ester.

The solid-phase procedure discussed above is well known in the art and has been essentially described by Stewart and Young, <u>Solid Phase Peptide Synthesis</u>, Freeman and Company, San Francisco, California (1969).

## FLOW DIAGRAM I

**Preparation of [D-Ala$^2$]-hpGRF(1-29)-$NH_2$**

BHA Resin + Boc-Arg(Tos)

- $(CH_3)_2CH-N=C=N-CH(CH_3)_2$
- $CH_2Cl_2$

} A

Boc-Arg(Tos-BHA Resin

- (a) $CH_2Cl_2$ wash
- (b) 33% TFA/$CH_2Cl_2$, twice
- (c) $CH_2Cl_2$ wash
- (d) EtOH wash
- (e) $CH_2Cl_2$ wash
- (f) $Et_3N/CHCl_3$, twice
- (g) $CH_2Cl_2$ wash

} B

Boc-Arg(Tos)-BHA Resin

(1) Boc-Ser(ØCH$_2$), A, B
(2) Boc-Met, A, B
(3) Boc-Ile, A, B
(4) Boc-Asp(ØCH$_2$), A, B
(5) Boc-Gln, A, B
(6) Boc-Leu , A, B
(7) Boc-Leu, A, B
(8) Boc-Arg (Tos), A, B
(9) Boc-Arg(Tos), A, B
(10) Boc-Ala, A, B
(11) Boc-Ser(/CH$_2$), A, B
(12) Boc-Leu, A, B
(13) Boc-Gln, A, B
(14) Boc-Gly, A, B
(15) Boc-Leu, A, B

FLOW DIAGRAM I (Continued)

(16) Boc-Val, A, B
(17) Boc-Arg (Tos), A, B
(18) Boc-Arg(Tos), A, B
(19) Boc-Tyr(4-Br/$CH_2OCO$), A, B
(20) Boc-Ser(Ø$CH_2$), A, B, MeOH wash, cycle B
(21) Boc-Asn, A, B
(22) Boc-Thr($OCH_2$), A, B
(23) Boc-Phe, A, B
(24) Boc-Ile, A, B
(25) Boc-Ala, A, B
(26) Boc-Asp(Ø$CH_2$), A, B
(27) Boc-D-Ala, A, B
(28) Boc-Tyr, A, B

↓

D-Ala[2]hpGRF(1-29)-BHA Resin

(a) HF, dimethylsulfide, p-cresol
(b) $Et_2O$ wash
(c) HOAc, Sephadex G-50 column chromatography
(d) Lyophilize
(e) Chromatography:octadecyl-silane silica, 15-50% $CH_3CN/H_2O$/0.1% TFA/80 psi
(f) Lyophilize

↓

[D-Ala$^2$]-hpGRF(1-29)-$NH_2$

To prepare formula (II) analogs, the C-terminal protected amino acid Boc-alanine is attached to the desired resin as described for preparing formula (I) peptides; likewise, to prepare formula (III) analogs, the C-terminal protected amino acid Boc-leucine is attached to the desired resin. The subsequent amino acid groups are then sequentially coupled to the solid phase in the manner described in Flow Diagram I.

To prepare a N-terminal alkanoyl [$R^1$ of (I), (II), and (III)] peptide, the peptide bonded to the resin is allowed to stir in 5 to 20% solutions of the appropriate acid anhydride in $CH_2Cl_2$ containing triethylamine or other standard acid accepting bases for 20 to 120 minutes at room temperature. Subsequently, the standard reagents for cleaving the peptide from the resin is used to obtain the desired peptide of formulas (I), (II), or (III).

Thus, by the above-mentioned procedure, the following peptides are prepared:

[D-$Tyr^{10}$]-hpGRF(1-29)$NH_2$
[D-$Ala^2$,D-$Tyr^{10}$]-hpGRF(1-29)$NH_2$
[D-$Ser^9$]-hpGRF(1-29)$NH_2$

[$Arg^{12,21}$]-hpGRF(1-29)-$NH_2$
[N-Acetyl,$Tyr^1$,$Arg^{12,21}$]-hpGRF(1-29)-$NH_2$
[D-$Ala^2$,$Arg^{12,21}$]-hpGRF(1-29)-$NH_2$
[D-$Ala^2$,$Arg^{12,21}$]-hpGRF(1-40)-$NH_2$
[N-Acetyl,D-$Ala^2$,$Arg^{12,21}$]-hpGRF(1-40)-$NH_2$
[D-$Ala^2$,$Arg^{12,21}$]-hpGRF(1-44)-$NH_2$
[N-Acetyl,$Agr^{12,21}$]-hpGRF(1-44)-$NH_2$

Accordingly, the present invention includes pharmaceutical compositions comprising at least one of the peptides of formulas (I), (II), or (III) as an active ingredient, in association with a pharmaceutical carrier or diluent for use in stimulating growth-hormone release in mammals as follows:

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R_2$;

(I)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-$R^2$

(II)

or

$R^1$-A-B-C-Ala-Ile-Phe-Thr-X-Q-Z-Arg-U-VAL-Leu-Gly-Gln-Leu-Ser-Ala-Arg-W-Leu-Leu-Gln-Asp-Ile-Y-Ser-Arg-Gln-Gln-Gly-Glu-Ser-Asn-Glu-Glu-Arg-Gly-Ala-Arg-Ala-Arg-Leu-$R^1$;

(III)

wherein $R^1$ is hydrogen or $C_1$-$C_6$ straight- or branched-chain alkanoyl; $R^2$ is $NR^3R^4$ or $OR^3$; $R^3$ and $R^4$ are selected from the group consisting of hydrogen and a straight- or branched-chain alkyl group containing one to six carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromoalanyl, 4-fluorophenylalanyl, D-4-fluorophenylalanyl, 4-methoxyphenylalanyl, D-4- methoxyphenylalanyl, 4-benzyloxyphenylalanyl, D-4- benzyloxyphenylalanyl, trytophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, D-5-methyltryptophyl or

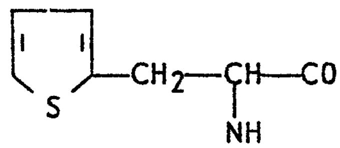
;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl or

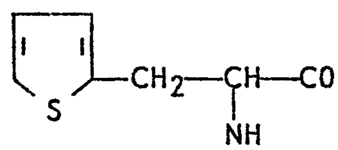
; and

C represents a member selected from aspartyl, D-aspartyl, glutamyl and D-glutamyl; Q is seryl or D-seryl; X represents asparaginyl or D-asparaginyl; Y represents norleucyl or methionyl, and Z is tyrosyl or D-tyrosyl; U is lysyl or arginyl; and W is lysyl or arginyl; provided that B is N-methylalanyl or N-methyl-D-alanyl; and/or Q is D-seryl; and/or Z is D-tyrosyl; and/or U and W are each arginyl; and the pharmaceutically acceptable salts thereof.

In practice, it has been found that the Formulas (I), (II), and (III) compounds of the present invention are effective for increasing the release of growth hormone in mammals when administered thereto in an amount sufficient to provide said-treated mammals with from 0.000001 to 0.1 mg/kg of mammalian body weight/ day of said Formulas (I), (II), or (III) compound.

Preferred peptides for increasing release of growth hormone in mammals have a structure selected from Formulas (I), (II), or (III) above, wherein $R^1$, $R^2$, $R^3$, and $R^4$ are selected from those described above; A is tyrosyl, D-tyrosyl, histidyl, D-histidyl; B is alanyl, D-alanyl, leucyl, D-leucyl; C is aspartyl, D-aspartyl, glutamyl, or D-glutamyl; Q is seryl or D-seryl; X is asparaginyl or D-asparginyl; Y is norleucyl or methionyl, and Z is tyrosyl or D-tyrosyl; and U and W are lysyl or arginyl; with the above-mentioned proviso; and the pharmaceutically acceptable salts thereof.

Another preferred group of compounds of the present invention effective for increasing growth hormone-release in mammals have the structure illustrated by Formula (I) wherein $R^1$is hydrogen or $C_1$-$C_3$ alkanoyl; $R^2$ is $NH_2$ or OH; A is tyrosyl, D-tyrosyl, or histidyl; B is alanyl or D-alanyl; C is aspartyl or D-aspartyl; Q is seryl or D-seryl; U is arginyl; W is arginyl; X is asparaginyl or D-asparginyl; Y is norleucyl or methionyl, and Z is tyrosyl or D-tyrosyl; and the pharmaceutically acceptable salts thereof.

The most preferred group of compounds of the invention have the Formula (I) configuration, wherein $R^1$ is selected from hydrogen or acetyl; $R^2$ is $NH_2$; A is selected from tyrosyl, D-tyrsyl or histidyl; B is alanyl or D-alanyl; C is aspartyl or D-aspartyl; Q is seryl or D-seryl; U is arginyl; W is arginyl; X is asparginyl or D-asparaginyl; and Y is norleucyl or methionyl; and the pharmaceutically acceptable salts thereof.

These peptides are useful for treatments of symptoms related to growth hormone deficiencies, for increasing wool growth, for increasing rate of growth of meat-producing animals, for improving carcass quality in meat-producing animals (i.e., more protein and less fat), for improving feed efficiency in meat-producing animals and dairy cows, for increasing milk production in dairy herds, and in healing wounds.

In order to facilitate a further understanding of the invention, the following examples are presented primarily for the purpose of illustrating certain more specific details thereof. The invention is not to be deemed limited thereby except as defined in the claims.

EXAMPLE 1

Protected D-tyrosine-10, human pancreatic growth hormone-releasing factor(9-29)-benzhydrylamine resin

Benzhydrylamine polystyrene resin (commercially available from Bachem, Inc., Torrance, California) (6.0 g, 3.00 mmol) in the chloride ion form is placed in the reaction vessel of a Beckman 990 automatic peptide synthesizer programmed to carry out the following work-wash cycle: (a) $CH_2Cl_2$; (b) 33% trifluoroacetic acid in $CH_2Cl_2$ (two times for one and 25 minutes each); (c) $CH_2Cl_2$; (d) $C_2H_5OH$; (e) $CH_2Cl_2$; (f) 10% $(C_2H_5)_3N$ in $CHCl_3$ (two times for two minutes each); and (g) $CH_2Cl_2$.

The neutralized resin is stirred with t-butyloxycarbonyl(Boc)-N-tosyl-l-arginine [Boc-Arg(Tos)] and diisopropylcarbodiimide (6 mmol) in $CH_2Cl_2$ for one hour, and the resulting amino acid resin is then cycled through the steps (a) through (g) in the above wash program. The following L-amino acids (3 mmol) are then coupled successively by the same reaction cycle: Boc-Ser(benzyl), Boc-Met, Boc-Ile, Boc-Asp(benzyl), Boc-Gln, Boc-Leu, Boc-Leu, Boc-Lys(4-chlorocarbenzoxy), Boc-Arg(Tos), Boc-Ala, Boc-Ser(benzyl), Boc-Leu, Boc-Gln, Boc-Gly, Boc-Leu, Boc-Val, Boc-Lys(4-chlorocarbenzoxy), Boc-Arg(tosyl), Boc-D-Tyr(4-bromocarbenzoxy) and Boc-Ser-(benzyl), except that Boc-Gln is coupled in the presence of 1-hydroxybenzotriazole (6 mmol) in dimethylformamide solution.

The completed peptide-benzhydrylamine resin, with the N-terminal Boc group removed, is then washed with $CH_3OH$ and air dried to give 11.79 g of material.

EXAMPLE 2

Preparation of protected D-tyrosine-10-human pancreatic-growth hormone-releasing factor(1-29)-benzhydrylamine resin

Peptide benzhydrylamine resin (0.98 g, 0.25 mmol) containing the 9-29 residues of the peptide as described in Example 1 is subjected to the work-wash cycle also

described in Example 1. The neutralized resin is stirred with Boc-L-asparagine (0.75 mmol), diisopropylcarbodiimide (0.75 mmol), and 1-hydroxybenzotriazole (0.75 mmol) in dimethylformamide.

The following amino acid derivatives (0.75 mmol) are then coupled successively by the same treatment cycle described in Example 1: Boc-Thr(benzyl), Boc-Phe, Boc-Ile, Boc-Ala, Boc-Asp(benzyl), Boc-Ala, and Boc-Tyr. The completed 1-29 peptide resin is then cycled through the standard work-wash program described in Example 17 in order to remove the N-terminal Boc group.

## EXAMPLE 3

**Preparation of D-tyrosine-10-human pancreatic growth hormone-releasing(1-29)-amine** [(D-$Tyr^{10}$)-hpGRF(1-29)-$NH_2$]

A mixture of the 1-29 peptide resin described in Example 18 (0.25 mmol) and a solution of hydrogen fluoride (10 mL), dimethylsulfide (26 mL), and p-cresol (4 mL) are stirred at 0°C for 75 minutes. Excess reagents are then rapidly evaporated under a stream of dry nitrogen and hydrogen fluoride (35 mL) is added, and the mixture stirred for a further 45 minutes at 0°C. Excess hydrogen fluoride is evaporated under nitrogen, and the resin plus free peptide are washed free of p-cresol with a large volume of diethyl ether.

The peptide is extracted into 50% acetic acid solution and applied to a column (2.5 x 95 cm) of Sephadex G-50 which is eluted with 2 M acetic acid. Eluant is monitored at 280 nM and fractions containing a major uv-absorbing peak are pooled and lyophilized. A solution of the lyophilized powder is eluted on a column (1.5 x 45 cm) of octadecylsilane silica having a mesh size of 15-20 M and pore size of 300 A° (purchased from Vydac, Hesperia, California). A linear elution gradient of 15-50% acetonitrile in 0.1% trifluoroacetic acid solution was employed at a pumping pressure of about 80 psi.

Emerging fractions are monitored at 280 nm and are each examined by analytical hplc at a wave length of 215 nm in order to ensure maximum homogeneity of pooled fractions. Lyophilization of these gave the title peptide as a white powder (32 mg).

This material gives one peak emerging at 38 minutes using analytical hplc on a column (0.4 x 25 cm) of Vydac octadecylsilane silica (5 M mesh size, 300 A°) which is pumped at 2 mL/min with a linear gradient of 20 to 40% acetonitrile in 0.1% trifluoracetic acid. Product yield is 75 mg from 0.25 mmole of starting material.

Hplc elution time = 27.9 minutes

Amino acid analysis gives: Asp, 31.0; Thr, 0.97; Ser, 2.81; Glu, 2.21; Gly, 105; Ala, 3.11; Val, 0.78; Met, 0.94; Ile, 1.70; Tyr, 2.00; Phe, 0.95; Lys, 1.85; Arg, 2.90.

## EXAMPLE 4

### Preparation of protected N-acetyl-tosyl-L-histidine-1,D-alanine-2,L-norleucine-27-human pancreatic growth hormone-releasing factor (1-29)-benzhydrylamine resin

Protected N-acetyl-tosyl-L-histidine-1,D-alanine-2,L-norleucine-27-human pancreatic growth hormone-releasing factor (1-29)-benzhydrylamine resin is prepared and in addition to placing L-tosyl-histidine in position 1 and D-alanine in position 2, L-norleucine is substituted for methionine in position 27, to yield the above-said benzhydrylamine resin.

## EXAMPLE 5

### Preparation of N-acetyl-L-histidine-1,D-alamine-2,L-norleucine-27-human pancreatic growth hormone-releasing (1-29)- amine

The N-acetyl-tosyl-L-histidine-1,D-alanine-2,L-norleucine-27-human pancreatic growth hormone-releasing

factor(1-29)-benzhydrylamine resin is treated with hydrogen fluoride, dimethyl sulfide and p-creasol. Excess reagents are removed from the misture by evaporation under a stream of dry nitrogen. The mixture is treated with hydrogen fluoride and excess hydrogen fluoride is again removed by evaporation under nitrogen. Diethyl ether is used to wash any free p-cresol from the resin and free peptide. Purification by the procedure of Example 3 yields: [(N-acetyl-$His^1$, D-$Ala^2$, $Nle^{27}$)-hpGRF(1-29)$NH_2$]. Product yield is 124 mg from 0.25 mmole of starting material. Hplc elution time (flow rate 1.5 mL minutes) is 31.2 minutes. Amino acid analysis gives: Asp, 3.24; Thr, 1.01; Ser, 2.97; Glu, 2.40; Gly, 1.11; Ala, 3.18; Val, 0.76; Ile, 1.63; Tyr, 1.03; Phe, 0.92; His, 1.08; Lys, 1.78; Arg, 2.99, Nle, 0.85.

## EXAMPLE 6

### Preparation of D-alanine-2,D-tyrosine-10-human pancreatic growth-releasing factor(1-29)-benzhydrylamine resin

Peptide-benzhydrylamine resin (0.25 mmol) prepared in Example 1 is subjected to the coupling cycles described in Example 2, except that D-alanine is used in place of L-alanine in position 2.

## EXAMPLE 7

### Preparation of D-alanine-2,D-tyrosine-10-human pancreatic growth hormone-releasing(1-29)-amine[(D-$Ala^2$,D-$Tyr^{10}$)-hpGRF(1-29)-$NH_2$]

The peptide resin (0.25 mmol) described in Example 6 is treated with hydrogen fluoride mixtures and purified as described in Example 3. The purified, lyophilized peptide weighs 130 mg. Hplc elution time is 28.3 minutes using the analytical Hplc conditions as described in Example 19. Amino acid analysis of a 6 M HCl hydrolysate gives the following amino acid ratios: Asp, 2.91; Thr, 0.97; Ser, 2.84; Glu, 2.20; Gly, 1.07; Ala, 3.00; Val, 0.96; Met, 0.59; Ile, 1.86; Leu, 4.08; Tyr, 1.86; Phe, 0.93; Lys, 1.99; Arg, 3.07.

EXAMPLE 8

Preparation of D-serine-9-human pancreatic growth hormone-releasing factor(1-29)-benzhydrylamine resin

Peptide-benzhydrylamine resin (0.25 mmole) prepared in Example 1, except that D-serine is used in place of L-serine in position 9, is subjected to the coupling cycles described in Example 2.

EXAMPLE 9

Preparation of D-serine-9-human pancreatic growth hormone-releasing(1-29)-amine[(D-$Ser^9$)-hpGRF(1-29)-$NH_2$]

The peptide resin (0.25 mmol) described in Example 8 is treated with hydrogen fluoride mixtures and purified as described in Example 3. The purified, lyophilized peptide weighs 108 mg. This material gives one peak emerging at 28.5 minutes using the analytical Hplc conditions as described in Example 3. Amino acid analysis of a 6 M HCl hydrolysate gives the following amino acid ratios: Thr, 0.95; Ser, 2.80; Glu, 2.16; Gly, 1.02; Ala, 3.04; Val, 0.90; Ile, 1.75; Leu, 4.00; Tyr, 1.91; Phe, 0.92; Lys, 2.06; Arg, 3.85; Met, 0.90.

EXAMPLE 10

Evaluation of peptide effects on growth hormone release in mammals using the rat as the test species

In this evaluation, the procedures described by W. A. Murphy et al., Endocrinology 109:491-495 (1980), were employed.

In growth hormone (GH) experiments, male rats (Charles Rivers) were anesthetized with NEMBUTAL® (6 mg per 100 g body weight) which also served to maintain stimulated plasma GH levels. Exactly 30 minutes after the rats were anesthetized, 0.5 mL of saline or the test peptide in saline was administered as a SC bolus. A 1 mL blood sample was drawn from the jugular vein 15 minutes after the injection of the peptide in saline. GH levels were determined using NIADDKD rat GH RIA components.

| Analog | Dose | Plasma GH | % Potency |
|---|---|---|---|
| D-Ser$^{9}$ | 16 | 1804 ± 312 (6) | 108 (77-151) |
| D-Ser$^{9}$ | 40 | 3652 ± 370 (6) | |
| D-Tyr$^{10}$ | 8 | 1674 ± 586 (5) | 228 (257-331) |
| D-Tyr$^{10}$ | 20 | 3975 ± 635 (5) | |
| N-AcHis$^{1}$, D-Ala$^{2}$, Nle$^{27}$ | 0.5 | 1146 ± 143 (6) | 1574 (1112-2226) |
| N-AcHis$^{1}$, D-Ala$^{2}$, Nle$^{27}$ | 1.25 | 1907 ± 603 (6) | |

hpGRF(1-29)-$NH_2$ Structure-Activity Studies

| Analog | Dose (μ/100 g BW) | Plasma GH (Ng/mL) |
|---|---|---|
| Saline | - | 27% $\pm$ 54 (11)* |
| [$Arg^{12,21}$]-hpGRF(1-29)-$NH_2$ | 10 | 688 $\pm$ 84 (8) |
| | 25 | 1613 $\pm$ 302 (8) |
| [N-Ac-Tyr,$Arg^{12,21}$]-hpGRF (1-29)-$NH_2$ | 1 | 796 $\pm$ 88 (8) |
| | 2.5 | 1649 $\pm$ 167 (8) |
| Saline | - | 309 $\pm$ 20 (6)* |
| [<u>D</u>-$Ala^2$,$Arg^{12,21}$]-hpGRF (1-29)-$NH_2$ | 0.4 | 755 $\pm$ 131 (6) |
| | 1.0 | 1752 $\pm$ 289 (6) |

* Number of rats in parenthesis

## EXAMPLE 11

**Preparation of arginine$^{12,21}$-human pancreatic growth hormone-releasing (1-29)-amide[$Arg^{12,21}$-hpGRF(1-29)-$NH_2$**

The peptide resin is prepared as above with L-arginine replacing D-lysine in positions 12 and 21 in the coupling cycle. This material is then coupled in the manner described in the flow diagram (I). The title product is then isolated by the usual manner.

The peptide resin is prepared as in Example 1 with L-arginine replacing D-lysine in positions 12 and 21 in the coupling cycle. This material is then coupled in the manner described in Example 2. The title product is then isolated by the usual manner.

EXAMPLE 12

Preparation of protected N-acetyl arginine$^{12,21}$-human pancreatic growth hormone-releasing factor (1-29)-amide [N-AcTyr,Arg$^{12,21}$-hpGRF(1-29)-$NH_2$

The title compound is prepared by the method described in the flow diagram (I) and purified by standard techniques.

EXAMPLE 13

Preparation of D-alanine$^2$, arginine$^{12,21}$-human growth hormone-releasing (1-29)-amide[(D-Ala$^2$,Arg$^{12,21}$)-hpGRF (1-29)-$NH_2$

The peptide-benzhydryl resin is prepared as described in flow diagram (I) with L-arginine replacing L-lysine in positions 12 and 21 of the coupling cycle. This material is coupled with D-alanine replacing L-alanine in position 2. The tile peptide is isolated and pufified in the usual manner.

EXAMPLE 14

Preparation of D-alanine-2-human pancreatic growth hormone-releasing (1-29)-carboxylic acid (D-Ala$^2$)-hpGRF (1-29)-OH.

Boc-Arg (Tos)(3mM) is coupled to the Merrifield hydroxymethyl resin by stirring 1,1'-carbonyldiimidazole (3mM) in a mixture of DMF and $CH_2Cl_2$ (10ml) with the blocked Arg at -5$^oC$ for 0.5 hr and then adding 1.5 mmol of the resin. The mixture is stirred for 20 hr. at room temperature and the resin is collected by filtration, washed with $CH_2Cl_2$, DMF, $CH_2Cl_2$, ETOH and $CH_2Cl_2$, and dried. The coupled resin mixture is further acetylated with a mixture of pyridine and $Ac_2O$ [20 ml, 1:1(v/v)] for 0.5 hr. to esterify any free hydroxymethyl resin and washed as before. The Boc-Arg(Tos)-resin is then coupled to the remaining requisite amino acids and further treated with HF and purified to afford the title compound.

## EXAMPLE 15

**Preparation of D-alanine$^2$, arginine$^{12,21}$-human growth hormone-releasing (1-29)-carboxylic acid (D-Ala$^2$,Arg$^{12,21}$)-hpGRF(1-29)-OH.**

In the manner described in Example 14, the title compound is prepared with L-arginine replacing L-lysine in positions 12 and 21 in the coupling cycle. The peptide is then purified in the usual manner.

## EXAMPLE 16

**Effect of D-Ala$^2$-GRF(1-29)$NH_2$ on Milk Production in Dairy Cows (Treatments Administered Subcutaneously)**

In this test, treatments are administered using a Latin Square design using four cows. Each treatment period is 10 days, and the interval between treatments is 4 days.

There is a pre-treatment period of one week, at the end of which each cow receives an indwelling jugular catheter. Catheters are flushed daily with heparinized (500-1000 IU/ml) sterile saline to help maintain potency and are replaced as needed.

The drugs are solubilized in sterile saline and injected subcutaneously each day with the dosage based on the cow's weight. Dosages range from 0.06 mg/kg animal/day to 60 mg/kg animal/day, preferably 0.10 mg/kg animal/day to 50 mg/kg/day.

On the first and last treatment days of each period, blood is collected at 15 minute intervals for 1 hour prior to and 1 1/2 hours after treatment; sampling continues at 30 minutes intervals for an additional 3 1/2 hours.

Milk samples are obtained on the evening proceeding each bleeding day, the morning of each bleeding day and combined (50% each).

Feed intake, refusals and milk production is monitored for all animals.

Results obtained are reported as mean milk production in kg/day for each treatment and compared against

a saline control.

| | D-Ala$^2$-GRF(1-29)$NH_2$ 0.4 mg/kg/day | D-Ala$^2$-GRF(1-29)$NH_2$ 0.8 mg/kg/day | Saline Control |
|---|---|---|---|
| Milk Production kg/day | 23.9 | 23.8 | 22.2 |
| % increase over control | +7.6% | +7.2% | - |

These results indicate that the growth hormone-releasing peptides of the present invention not only elevate growth hormone release but that the biological activity of increasing milk production in the subject animals is observed. This, of course, indicates that other biological responses, such as increased meat production, increasing growth rate, increasing wool production, etc. result by the administration of the present peptides to animals.

WHAT IS CLAIMED IS:

1. A peptide characterized by: $R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R^2$;

(I)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-$R^2$;

(II)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-Arg-Ala-Arg-Leu-$R^2$;

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

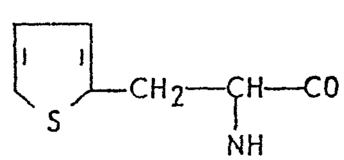 ;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

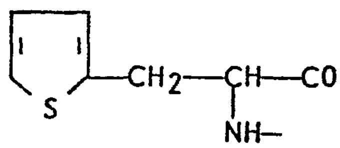 ; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or U and W are each arginyl; and the pharmaceutically acceptable salts thereof.

2. A peptide according to Claim 1, wherein $R^1$ is selected from hydrogen or $C_1$-$C_3$ alkanoyl; $R^2$ is $NH_2$ or OH; A is tyrosyl, D-tyrosyl, histidyl; B is alanyl or D-alanyl; C is aspartyl or D-aspartyl; Q is seryl or D-seryl; U is arginyl; W is arginyl; X is asparaginyl or D-asparaginyl; Y is norleucyl or methionyl, and Z is tyrosyl or D-tyrosyl; and the pharmaceutically acceptable salts thereof.

3. A peptide according to Claim 1, having the Formula (I) structure wherein $R^1$ is selected from hydrogen or acetyl; $R^2$ is $NH_2$ or OH; A is tyrosyl, D-tyrosyl, or histidyl; B is alanyl or D-alanyl; C is asparatyl or

D-asparatyl; Q is seryl or D-seryl; U and W are each arginyl; X is asparaginyl or D-asparaginyl; Y is norleucyl or methionyl, and Z is tyrosyl or D-tyrosyl; and the pharmaceutically acceptable salts thereof.

4. The peptide according to Claim 1, said peptide characterized by: Ac-His-D-Ala-Asp-Ala-Ile-Phe-Thr-Asn-Ser-D-Tyr-Arg-Lys-Val-Leu-Gly-Gln-Leu-Ser-Ala-Arg-Lys-Leu-Leu-Gly-Asp-Ile-Nle-Ser-Arg-$NH_2$; Tyr-Ala-Asp-Ala-Ile-Phe-Thr-Asn-Ser-D-Tyr-Arg-Lys-Val-Leu-Gly-Gln-Leu-Ser-Ala-Arg-Lys-Leu-Leu-Gln-Asp-Ile-Met-Ser-Arg-$NH_2$; Tyr-D-Ala-Asp-Ala-Ile-Phe-Thr-Asn-Ser-D-Tyr-Arg-Lys-Val-Leu-Gly-Gln-Leu-Ser-Ala-Arg-Lys-Leu-Leu-Gln-Asp-Ile-Met-Ser-Arg-$NH_2$; Tyr-Ala-Asp-Ala-Ile-Phe-Thr-Asn-D-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$; Tyr-D-Ala-Asp-Ala-Ile-Phe-Thr-Asn-Ser-Tyr-Arg-Arg-Val-Leu-Gly-Gln-Leu-Ser-Ala-Arg-Arg- Leu-Leu-Gln-Asp-Ile-Met-Ser-Arg-$NH_2$ and its pharmeceutically acceptable salts; Tyr-Ala-D-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; D-Tyr-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Ac-Tyr-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceitucally acceptable salts; Ac-D-Tyr-D-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Tyr-D-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-OH and its pharmaceutically acceptable salts; or Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-OH and its pharmáceutically acceptable salts.

5. A method for increasing the release of growth hormone in mammals, said method characterized by:

administering thereto from 0.000001 to 0.1 mg/kg of mammalian body weight/day of a peptide having the formula,

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R^2$;

(I)

or

$R^1$-A-B-C-Ala-Ile-Phe-Thr-X-Q-Z-Arg-W-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Agr-Gly-Ala-$R^2$;

(II)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-Arg-Ala-Arg-Leu-$R^2$;

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl,

D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

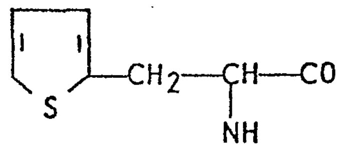 ;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

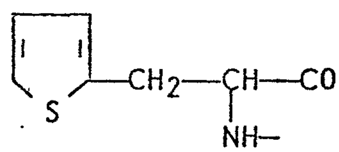 ; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or U and W are each arginyl; and the pharmaceutically acceptable salts thereof.

6. A method according to Claim 5 wherein $R^1$ is selected from hydrogen or $C_1$-$C_3$ alkanoyl; $R_2$ is $NH_2$ or OH; A is tyrosyl, D-tyrosyl, or histidyl; B is alanyl or D-alanyl; C is aspartyl or D-aspartyl; Q is seryl or D-seryl; U is arginyl; W is arginyl; X is asparaginyl or D-asparaginyl; Y is nor leucyl or methionyl, and Z is tyrosyl or D-tyrosyl; and the pharmaceutically acceptable salts thereof.

7. A method according to Claim 5, wherein the peptide is of the Formula (I); $R^1$ is hydrogen or acetyl; $R^2$ is $NH_2$; A is tyrosyl, D-tyrosyl, or histidyl; B is alanyl or D-alanyl; C is aspartyl or D-aspartyl; Q is seryl or D-seryl; U and W are each arginyl; X is asparaginyl or D-asparaginyl; Y is norleucyl or methionyl, and Z is tyrosyl or D-tyrosyl; and the pharmaceutically acceptable salts thereof.

8. A method according to Claim 5, wherein the peptide is: Tyr-D-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Tyr-Ala-D-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; D-Tyr-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Ac-Tyr-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Ac-D-Tyr-D-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-$NH_2$ and its pharmaceutically acceptable salts; Tyr-D-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Met-Ser-Arg-OH and its pharmaceutically acceptable salts; or Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-OH and its pharmaceutically acceptable salts.

9. A process for the preparation of a peptide having the formula, $R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R^2$;

(I)

or

$R^1$-A-B-C-Ala-Ile-Phe-Thr-X-Q-Z-Arg-U-Val-Leu-Gly-Gln-Leu-Ser-Ala-Arg-Lys-Leu-Leu-Gln-Asp-Ile-Y-Ser-Arg-Gln-Gln-Gly-Glu-Ser-Asn-Gln-Glu-Arg-Gly-Ala-$R^2$;

(II)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-Arg-Ala-Arg-Leu-$R^2$;

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenyl-D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

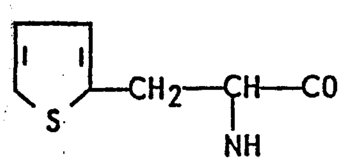 ;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl,

tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

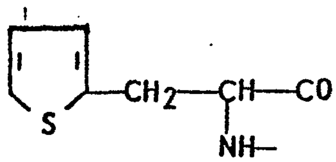

; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or U and W are each arginyl; and the pharmaceutically acceptable salts thereof; said method characterized by: a C-terminal protected amino acid to a resin in the presence of a coupling agent; washing with an inert solvent; deprotecting in the presence of an acid; repeating this sequence with the subsequent protected amino acids as defined in formula (I), (II) and (III), stepwise, from the C-terminus of the peptide; and finally detaching the peptide from the resin the presence of an acid to afford the peptide of formula (I), (II) and (III) wherein $R^2$ is $NH_2$, wherein said resin is BHA or p-ME-BHA; or detaching the peptide from the resin with ammonia, alkylamine or dialkylamine and deprotecting with an acid to afford the peptide of formula (I), (II), and (III), wherein $R^2$ is $NR^3R^4$, wherein said resin is a chlormethylated or hydroxymethylated resin; or detaching the peptide from the resin in the presence of an acid to afford the peptide of the formula (I), (II) or (III) wherein $R^2$ is OH; wherein said resin is a chloromethylated or hydroxymethylated resin; or detaching the peptide from the resin in the presence of a base and an $R^3$OH alcohol, followed by acid treatment to afford the peptide of the formula (I), (II) or (III), wherein $R^2$ is $OR^3$, wherein $R^3$ is a straight- or branched chain alkyl group containing one to six carbon atoms and said resin is a chloromethylated or hydroxymethylated resin.

10. A method for increasing milk production in dairy cows, said method characterized by: administering to said cows an amount of a formula I, II or III peptide sufficient to increase milk production in said cows, said peptide having a formula: $R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R^2$;

(I)

or

$R^1$-A-B-C-Ala-Ile-Phe-Thr-X-Q-Z-Arg-U-Val-Leu-Gly-Gln-Leu-Ser-Ala-W-Leu-Leu-Gln-Asp-Ile-Y-Ser-Arg-Gln-Gln-Gyl-Glu-Ser-Asn-Gln-Glu-Arg-Gly-Ala-$R^2$;

(II)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-Arg-Ala-Arg-Leu-$R^2$;

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl,

D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

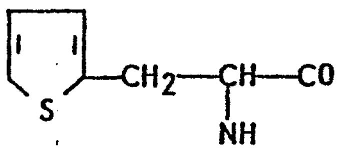 ;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

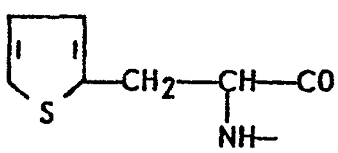 ; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or U and W are each arginyl; and the pharmaceutically acceptable salts thereof.

11. A method for increasing the growth rate of animals, said method characterized by: administering to said animals a growth promoting amount of a formula I, II or III peptide having a formula: $R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-$R^2$;

(I)

or

$R^1$-A-B-C-Ala-Ile-Phe-Thr-X-Q-Z-Arg-U-Val-Leu-Gly-Gln-Leu-Ser-Ala-W-Leu-Leu-Gln-Asp-Ile-Y-Ser-Arg-Gln-Gln-Gyl-Glu-Ser-Asn-Gln-Glu-Arg-Gly-Ala-$R^2$;

(II)

or

$R^1$-A-B-C-Ala-Ile-Phe-Thr-X-Q-Z-Arg-U-Val-Leu-Gly-Gln-Leu-Ser-Ala-Arg-W-Leu-Leu-Gln-Asp-Ile-Y-Ser-Arg-Gln-Gln-Gly-Glu-Ser-Asn-Gln-Glu-Arg-Gly-Ala-Arg-Ala-Arg-Leu-$R^2$;

(III)

Wherein $R^1$ is hydrogen or $C_1$-$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1-6 carbon atoms; A represents a member selected from tyrosyl, D-tyrosyl, histidyl, D-histidyl, phenylalanyl, D-phenylalanyl, 4-chlorophenylalanyl, D-4-chlorophenylalanyl, 4-bromophenylalanyl, D-4-bromophenylalanyl, 4-fluorophenylalanyl, D-4-fluorophenylalanyl, 4-methoxyphenylalanyl, D-4-methoxyphenylalanyl, 4-benzyloxyphenylalanyl,

D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromotryptophyl, D-5-bromotryptophyl, 5-methoxytryptophyl, D-5-methoxytryptophyl, 5-methyltryptophyl, or D-5-methyltryptophyl or

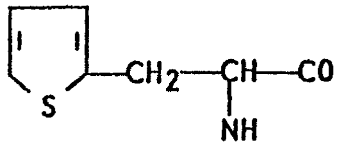

;

B represents a member selected from alanyl, D-alanyl, N-methylalanyl, N-methyl-D-alanyl, leucyl, D-leucyl, phenylalanyl, D-phenylalanyl, 4-chlorophenylalanyl, D-4-chlorophenylalanyl, 4-bromophenylalanyl, D-4-bromophenylananyl, 4-fluorophenylalanyl, D-4-fluorophenylalanyl, 4-methoxyphenylalanyl, D-4-methoxyphenylalanyl, 4-benzyloxyphenylalanyl, D-4-benzyloxyphenylalanyl, tryptophyl, D-tryptophyl, 5-fluorotryptophyl, D-5-fluorotryptophyl, 5-chlorotryptophyl, D-5-chlorotryptophyl, 5-bromtryptophyl, D-5-bromotryptophyl, 5-methoxytryptophyl, D-5-methoxytryptophyl or

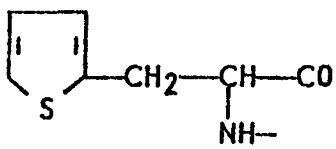

; and

C represents a member selected from aspartyl, D-aspartyl, glutamyl and D-glutamyl; Q is seryl or D-seryl; X is asparaginyl or D-asparginyl; Y is norleucyl or methionyl, Z is tyrosyl or D-tyrosyl; U is lysyl or arginyl; and W is lysyl or arginyl; provided that at least one of these conditions apply: B is N-methylalanyl or N-methyl-D-alanyl; and/or Q is D-seryl; and/or Z is D-tyrosyl; and/or U and W are each arginyl; and the pharmaceutically acceptable salts thereof.